# EUROPEAN PATENT APPLICATION

(11) **EP 4 019 074 A1**
(43) Date of publication of application: **29.06.2022**
(21) Application number: 20217461.1
(22) Date of filing: 28.12.2020
(51) Int. Cl.: A61M 25/09, A61B 34/30, A61B 34/00

(54) **CATHETER ROBOT**

(71) Applicant: Robocath, 76000 Rouen (FR)
(72) Inventor: FOURNIER, Bruno, 41100 SAINT OUEN (FR)
(74) Representative: Plasseraud IP

(57) **Abstract**

This invention relates to a catheter robot comprising: a base (40), at least 3 catheter translation modules (1, 2, 3) supported by said base (40), each catheter translation module (1, 2, 3) comprising two pairs of movable pads (20, 22, 23, 24, 25): said pads of a same pair at least partly facing each other, alternatively, each of said two pairs clamping between its pads a flexible elongated medical element (11, 12, 13, 14, 15) so as to then translate longitudinally said flexible elongated medical element, said catheter translation modules (1, 2, 3) being successively longitudinally disposed so that: first catheter translation module (1) has its pairs of pads (20) performing a longitudinal translation of a first flexible elongated medical element which is a catheter guide (11), second catheter translation module (2): can translate longitudinally with respect to said first catheter translation module (1), its pairs of pads (22, 24) performing a longitudinal translation of a second flexible elongated medical element which is a catheter (12, 14), preferably a catheter balloon or a catheter stent, third catheter translation module (3): can translate longitudinally with respect to said first catheter translation module (1), its pairs of pads (23, 25) performing a longitudinal translation of a third flexible elongated medical element which is a guide (13, 15) of said catheter.

## Description

### FIELD OF THE INVENTION

The invention relates to the technical field of catheter robots, and especially to the technical field of catheter robots including several flexible elongated medical elements.

### BACKGROUND OF THE INVENTION

A catheter robot has to perform translations of several flexible elongated medical elements. Those flexible elongated medical elements can include a catheter guide partially surrounding a catheter itself partially surrounding a guide of a catheter. Those translations have to be independent from one another, since those flexible elongated medical elements have to move independently from one another, at least sometimes. However, at some other times, at least some of those flexible elongated medical elements may have to move synchronously with one another.

According to a prior art, each flexible elongated medical element is translated by a translation device, most often one or more pairs of rollers, sometimes a pair of pads facing each other and clamping this flexible elongated medical element in between.

Therefore, when those flexible elongated medical elements have to move independently from one another, each translation device performs translation motion of its associated flexible elongated medical element independently from one another translation device.

However, when those flexible elongated medical elements have to move synchronously from one another, which means not necessarily the same translation moves, but at least different translation moves which have to be synchronized together, each translation device performs translation motion of its associated flexible elongated medical element while trying and synchronizing this translation motion with the other translation motions performed by the other translation devices for the other flexible elongated medical elements.

This prior art presents an inconvenient of needing to synchronize two or three, or even more, translation motions respectively of two or three, or even more, translation devices, what can become rather complex, especially when some of these translation motions are triggered by specific variable inputs of user, including variations of speed and directions.

### SUMMARY OF THE INVENTION

The object of the present invention is to alleviate at least partly the above-mentioned drawbacks.

More particularly, the invention aims at providing for a global translation system which can have a good compromise between simplicity and efficiency, not only in situations when one or more flexible elongated medical elements have to translate independently from each other or from one another, but also when one or more flexible elongated medical elements have to translate in synchronization with each other or with one another.

According to the invention, this problem, in case of those flexible elongated medical elements needing to move synchronously with one another, is solved by splitting the translation motion into two parts:
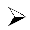 a first part of "synchronized" translation motion where one or more translation devices translate themselves, as complete devices, i.e. as sliding supports, to match the synchronization with another translation motion of another flexible elongated medical device with which synchronization is to be done,
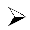 while a second part of translation motion, matches the specific user inputs, by performing corresponding relative translation(s) of one or more flexible elongated medical elements with respect to their own one or more translation devices, so as to take into account, besides the needed synchronization between translation devices, the specific additional user inputs, still different, at least possibly, for each flexible elongated medical element.

According to the invention, in some cases, the translation motion, of at least some of the flexible elongated medical elements, can be performed by two different manners:
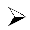 either by translating the translation device itself, with no relative translation between this translation device and its associated flexible elongated medical element,
   ∘ this first translation motion corresponding to the need of synchronization with the translation motion of another associated flexible elongated medical element of another translation device,
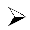 and/or by translating this associated flexible elongated medical element relatively to its associated translation device,
   ∘ this second translation motion corresponding to the specific variable user input corresponding to the specific translation wanted by the user.

According to the invention, these separate and independent translation devices, being able to translate relatively to each other, at least for some of them, will be therefore called translation modules, for all of them. These separate and independent translation devices also have each an internal translation mechanism able to translate their respectively associated flexible elongated medical elements, without translating themselves, while remaining themselves stationary with respect to the base, i.e. to the body of the catheter robot.

This object is achieved with a catheter robot comprising: a base, at least 3 catheter translation modules supported by said base, each catheter translation module comprising two pairs of movable pads: said pads of a same pair at least partly facing each other, alternatively, each of said two pairs clamping between its pads a flexible elongated medical element so as to then translate longitudinally said flexible elongated medical element, said catheter translation modules being successively longitudinally disposed so that: first catheter translation module has its pairs of pads performing a longitudinal translation of a first flexible elongated medical element which is a catheter guide, second catheter translation module: can translate longitudinally with respect to said first catheter translation module, its pairs of pads performing a longitudinal translation of a second flexible elongated medical element which is a catheter, preferably a catheter balloon or a catheter stent, third catheter translation module: can translate longitudinally with respect to said first catheter translation module, its pairs of pads performing a longitudinal translation of a third flexible elongated medical element which is a guide of said catheter.

This object may also be achieved with a catheter robot comprising: a base, at least 3 catheter translation modules supported by said base, each catheter translation module comprising an internal translation mechanism structured and disposed so as to translate longitudinally a flexible elongated medical element, said catheter translation modules being successively longitudinally disposed so that: first catheter translation module has its internal translation mechanism performing a longitudinal translation of a first flexible elongated medical element which is a catheter guide, second catheter translation module: can translate longitudinally with respect to said first catheter translation module, its internal translation mechanism performing a longitudinal translation of a second flexible elongated medical element which is a catheter, preferably a catheter balloon or a catheter stent, third catheter translation module: can translate longitudinally with respect to said first catheter translation module, its internal translation mechanism performing a longitudinal translation of a third flexible elongated medical element which is a guide of said catheter.

Preferably, not only translation motion can be freely and independently imparted to all flexible elongated medical elements, but also rotation motion, all the more than the specific internal translation structure of the translation modules, can also allow for imparting rotation move too of its associated flexible elongated medical element.

Preferred embodiments comprise one or more of the following features, which can be taken separately or together, either in partial combination or in full combination.

Preferably, the catheter robot comprises a control unit configured for commanding the translation of the second catheter translation module by following the translation of the first flexible elongated medical element, and for controlling the translation of the second flexible elongated medical element by compensating the translation of the second catheter translation module so as to maintain the second flexible elongated medical element stationary with respect to said base.

Hence, when a flexible elongated medical element is translated by a translation module, and when the translation module of another flexible elongated medical element follows this translation, so as to always keep a certain distance with this former translation module, and so as to avoid collision between translation modules, and when at the same time, this another flexible elongated medical element does not need to be translated, then this another flexible elongated medical element can be still kept at the same location, by imparting to him two opposite translation motions which compensate exactly for each other, the translation motion of its associated translation module in a first direction and the relative translation motion of this another flexible elongated medical element with respect to its associated translation module in a second direction, parallel and opposite to the first direction, both translation motions having equal intensities.

Preferably, said catheter translation modules are all structurally identical.

Hence, not only can translation motion of all flexible elongated medical elements be performed independently, but also this independence can be obtained with a rather simple and cheap system, by making this system fully modular.

Preferably, said catheter translation modules are successively longitudinally spaced apart from one another.

Hence, this leads to an improved supplementary degree of liberty between flexible elongated medical elements, because thereby these flexible elongated medical elements can perform more easily independent translations moves in both opposite directions of translation line, and with extended translation travels, even in an over the wire configuration.

Preferably, said third catheter translation module can translate longitudinally with respect both to said first catheter translation module and to said second catheter translation module.

Hence, this leads to a supplementary degree of liberty between second and third flexible elongated medical elements, because thereby these flexible elongated medical elements can perform independent translations moves, even in an over the wire configuration.

Preferably, a first Y connector is located between said first and second catheter translation modules, one of Y branches and output of Y being disposed longitudinally, a second Y connector is located between said second and third catheter translation modules, one of Y branches and output of Y being disposed longitudinally.

Hence, other flexible elongated medical elements or materials can be added sideways so as to afterwards translate in parallel to the other flexible elongated medical elements already in line.

Preferably, said guide of catheter translates within said catheter itself translating within said catheter guide, for at least part of their respective lengths.

Hence, this translation modular system can also work with an over the wire configuration.

Preferably, the catheter robot comprises a control unit configured for commanding the translation of the third catheter translation module by following the translation of the second flexible elongated medical element, and for controlling the translation of the third flexible elongated medical element by compensating the translation of the third catheter translation module so as to maintain the third flexible elongated medical element stationary with respect to said base.

Hence, when a flexible elongated medical element is translated by a translation module, and when the translation module of another flexible elongated medical element follows this translation, so as to always keep a certain distance with this former translation module, and so as to avoid collision between translation modules, and when at the same time, this another flexible elongated medical element does not need to be translated, then this another flexible elongated medical element can be still kept at the same location, by imparting to him two opposite translation motions which compensate exactly for each other, the translation motion of its associated translation module in a first direction and the relative translation motion of this another flexible elongated medical element with respect to its associated translation module in a second direction, parallel and opposite to the first direction, both translation motions having equal intensities.

Preferably, said second and third catheter translation modules are fastened together so as to be able to translate longitudinally with respect to said first catheter translation module only jointly.

Hence, the global translation system is made simpler, since two translation modules are linked together so as to translate as if they were a single translation module.

Preferably, said pairs of pads of said third catheter translation module are transversely shifted from said pairs of pads of said second catheter translation module.

Hence, this translation modular system can also work with a rapid exchange configuration.

Preferably, said pairs of pads of said third catheter translation module are transversely shifted from said pairs of pads of said second catheter translation module, so that: said guide of catheter and said catheter can translate: parallel to each other over the length extending along said 3 catheter translation modules, along each other for most of the length of said catheter, both within said catheter guide for at least part of their respective lengths, said guide of catheter translating within said catheter for only distal portions of their respective lengths.

Hence, this shift is a simple and efficient way to allow for a rapid exchange configuration, with a simpler structure of the global translation system, since two translation modules are linked together so as to translate as if they were a single translation module.

Preferably, said first catheter translation module is fixed with respect to said base.

Hence, this first catheter translation module can be considered as a "master" translation module, other translation modules being "slave" translation modules when they translate themselves so as to keep synchronization with the translation move of the first flexible elongated medical element associated to this first translation module. Thereby, not only can independence of all flexible elongated medical elements be maintained, but the global translation system is made simpler, since this first translation module can be kept fixed and immobile with respect to the base of the whole catheter robot.

Preferably, said base is an end of an articulated robotic arm.

Hence, this group of three translation modules can be set at any desired position with respect to the patient.

Preferably, said catheter translation modules are successively longitudinally disposed from one another, in an order which is: first then second and then third catheter translation module.

Hence, this is more practical to move independently all flexible elongated medical elements, while keeping the biggest of them with the shorter length.

Preferably, the catheter robot comprises at least 4 catheter translation modules supported by said base, which are successively longitudinally disposed.

Hence, the number and the type of configurations which can be used with the catheter robot of the invention, is richer and broader.

An example of such a catheter robot is a catheter robot where:
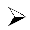 said first translation module translates a catheter guide,
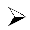 said second translation module translates a catheter,
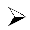 said third translation module translates a micro-catheter,
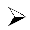 said fourth translation module translates a guide of catheter.

Preferably, the catheter robot comprises at least 5 catheter translation modules supported by said base, which are successively longitudinally disposed, and preferably only 5 catheter translation modules.

Hence, the number and the type of configurations which can be used with the catheter robot of the invention, is richer and broader.

Another example of such a catheter robot is a catheter robot where:
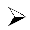 said first translation module translates a catheter guide,
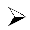 said second translation module translates a first balloon or stent,
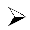 said third translation module translates a first guide of catheter,
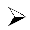 said fourth translation module translates a second balloon or stent,
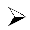 said fifth translation module translates a second guide of catheter.

Preferably, second and third catheter translation modules are configured as rapid exchange catheter translation modules, fourth and fifth catheter translation modules are configured as over the wire catheter translation modules.

Hence, the number and the type of configurations which can be used with the catheter robot of the invention, is richer and broader.

An application for this type of catheter robot could be CTO (Chronical Total Occlusion), where both types of catheters, rapid exchange type and over the wire type, are mixed within a same and single catheter robot.

Preferably, all second to fifth catheter translation modules are configured as over the wire catheter translation modules.

Hence, the number and the type of configurations which can be used with the catheter robot of the invention, is richer and broader.

Preferably, each catheter translation module is also a catheter rotation module: alternatively, each of said two pairs clamping between its facing pads a flexible elongated medical element so as to then rotate around longitudinal axis said flexible elongated medical element, by making said flexible elongated medical element rolling between its facing pads.

Hence, not only translation motion can be freely and independently imparted to all flexible elongated medical elements, but also rotation motion, all the more than the specific structure of the translation modules, with a pair of pads facing each other and clamping its associated flexible elongated medical element, allows for imparting rotation move too of its associated flexible elongated medical element.

Further features and advantages of the invention will appear from the following description of embodiments of the invention, given as non-limiting examples, with reference to the accompanying drawings listed hereunder.

### BRIEF DESCRIPTION OF THE DRAWINGS

Fig. 1 shows schematically an over the wire configuration of flexible elongated medical elements, with a manual control.
Fig. 2 shows schematically a rapid exchange configuration of flexible elongated medical elements, with a manual control.
Fig. 3 shows schematically a specific problem happening in an over the wire configuration of flexible elongated medical elements, with a manual control.
Fig. 4 shows schematically an example of a first embodiment of a catheter robot according to the invention, with an application to an over the wire configuration of flexible elongated medical elements.
Fig. 5 shows schematically an example of a second embodiment of a catheter robot according to the invention, with an application to a rapid exchange configuration of flexible elongated medical elements.

### DETAILED DESCRIPTION OF THE INVENTION

In the vascular field, there are mainly two types of catheter configurations, which are first the over the wire (OTW) catheter configuration and second the rapid exchange (REX) catheter configuration.

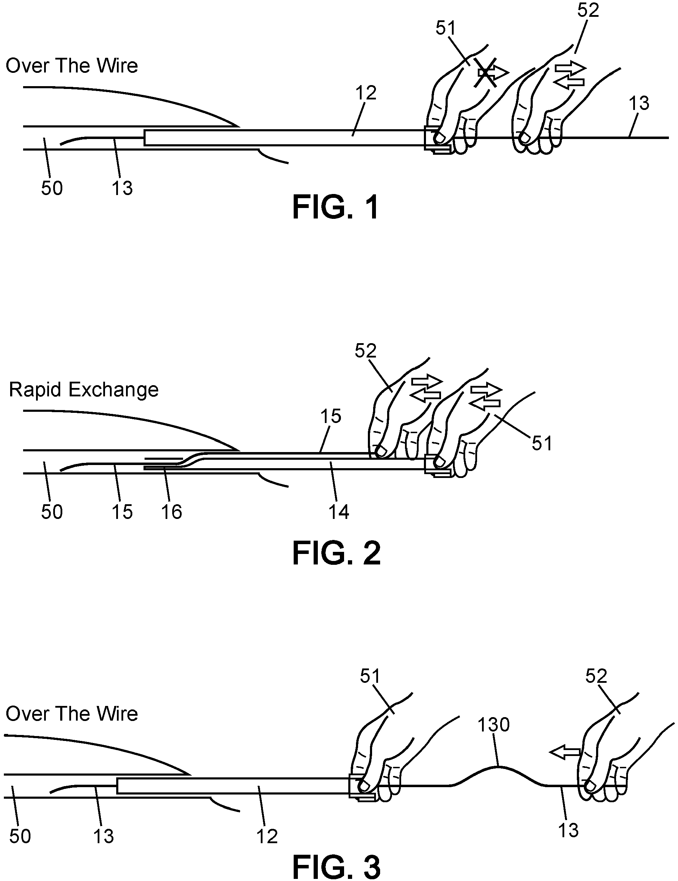

Fig. 1 shows schematically an over the wire configuration of flexible elongated medical elements, with a manual control.

A first hand 51 of practitioner is manipulating a catheter 12, and especially translating the catheter 12 along its own axis. A second hand 52 of practitioner is manipulating a guide 13 of catheter, and especially translating the guide 13 of catheter along its own axis. The catheter 12 surrounds the guide 13 of catheter on part of its length, the catheter 12 being shorter than the guide 13 of catheter. The guide 13 of catheter can slide within the catheter 12. Guide 13 of catheter and catheter 12 are coaxial. First hand 51 can translate catheter 12 forward and backward. Second hand 52 can translate guide 13 of catheter forward and backward. First hand 51 can translate catheter 12 independently from second hand 52 translating guide 13 of catheter. Catheter 12 and guide 13 of catheter are both inserted within patient, and more precisely within a vein 50 or an artery 50 of patient.

The main difference lies in the handling. In the case of an OTW assembly, if the practitioner wishes to move the catheter 12 back, then the second hand 52 which handles the guidewire 13 has also to move back, because the grip area available on the guidewire 13 has been reduced by the retraction length of the catheter 12. If the practitioner wants to remove the catheter 12 without changing the position of the guidewire 13 in the vein 50 or artery 50 of the patient, what is indeed a rather frequent case in intervention, it is needed to have a guidewire 13 at least twice as long as the catheter 12, so that at any time a guidewire 13 portion is accessible to the practitioner's hand, upstream or downstream of the catheter 12, in order to hold the guidewire 13 and prevent it from moving in the vein 50 or artery 50 of the patient.

Fig. 2 shows schematically a rapid exchange configuration of flexible elongated medical elements, with a manual control. Rapid exchange configuration distinguishes mainly from over the wire configuration by the fact that the coaxial part 16 of the catheter 14 and of the guide 15 of the catheter is very short, and in practice is often limited to a few centimeters at distal end of catheter 14. Throughout the rest of its length, the guidewire 15, which is the guide 15 of catheter 14, is therefore outside, parallel to the catheter 14.

A first hand 51 of practitioner is manipulating a catheter 14, and especially translating the catheter 14 along its own axis. A second hand 52 of practitioner is manipulating a guide 15 of catheter, and especially translating the guide 15 of catheter along its own axis. The catheter 14 surrounds the guide 15 of catheter on a very short part 16 of its length, this very short part 16 being located at distal end of catheter 14. On most of their respective lengths, catheter 14 and guide 15 of catheter are outside from each other, and parallel to each other. The guide 15 of catheter can slide within very short part 16 of catheter 14. Guide 15 of catheter and catheter 14 are coaxial only within very short part 16. First hand 51 can translate catheter 14 forward and backward. Second hand 52 can translate guide 15 of catheter forward and backward. First hand 51 can translate catheter 14 independently from second hand 52 translating guide 15 of catheter. Catheter 14 and guide 15 of catheter are both inserted within patient, and more precisely within a vein 50 or an artery 50 of patient.

In contrast, the rapid exchange configuration (also called quick exchange configuration) allows for the guidewire 15 and for the catheter 14 to be manipulated without interference between each other, and the hands 51 and 52 can stay in the same place, assuming that translation is performed using small repeated steps. For example, push the flexible elongated medical element, either guidewire 15 or catheter 14, by a small distance, for example by 1 cm, from proximal position to distal position, i.e. from right to left taking the disposition of figure 2 into account, release it, replace the fingers of the hands 51 and 52 at their original position, grip the flexible elongated medical element, either guidewire 15 or catheter 14, again and repeat the same steps of this cycle several times. In this way, if those 1 cm back and forth movements are neglected, the average positions of the hands 51 and 52 remain rather unchanged. This manipulating principle has been used in a robotic module a described in the patent application FR3022147 (hereby incorporated by reference), with the same assignee as present patent application.

Fig. 3 shows schematically a specific problem happening in an over the wire configuration of flexible elongated medical elements, with a manual control.

Even if the second hand 52 manipulating the guidewire 13 could be placed farther from the proximal end (convention: proximal = close to the physician / distal = close to the patient) of the catheter 12, pushing the guidewire 13 from that distance would cause the guidewire 13 to bend rather than to translate inside the catheter 12. This can be seen on figure 3, with the bending 130 of the guidewire 13.

Because of their respective strengths and weaknesses, these two solutions are rather dedicated respectively to specific applications. In interventional cardiology, the catheters 14 used are mainly REX. In interventional radiology, for instance lower and upper limbs, neurology, etc., OTW catheters 12 are, on the contrary, generally preferred.

To be able to build a robot that handle both OTW and REX catheters, following features are interesting:
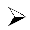 the considered translation module, which can translate and/or rotate a flexible elongated medical element (a guidewire, a balloon, a stent, etc.), should be able to reproduce the function of a hand 51 or 52 as shown on both figures 1 and 2.
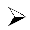 the considered translation module should be duplicated according to the number of flexible elongated medical elements manipulated. For example, to robotize the manipulation performed on either figure 1 or on figure 2, two such translation modules would be needed.
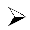 The considered translation module should be located at the most possible distal position in order to avoid the negative effect shown on figure 3.

For two rapid exchange flexible elongated medical elements, the two corresponding translation modules should be fixed, relatively to the patient and relatively to the base of the catheter robot.

For two over the wire flexible elongated medical elements, the catheter translation module should be fixed relatively to the patient and relatively to the base of the catheter robot, whereas the guidewire translation module should move back and forth in order to follow the translation motion of the catheter.

Therefore, designing a catheter robot for the manipulation of REX flexible elongated medical elements should not seem, at first sight, to lead to the same architecture as a robot for OTW flexible elongated medical elements.

One purpose of embodiments of the invention is to try and provide for, if not a unique architecture, or at least very close and quite similar architectures, which can accommodate either OTW or REX flexible elongated medical elements. Two embodiments families can exist, within which either the same robot can manipulate both types of flexible elongated medical elements, or the same technology is implemented in two slightly different catheter robots.

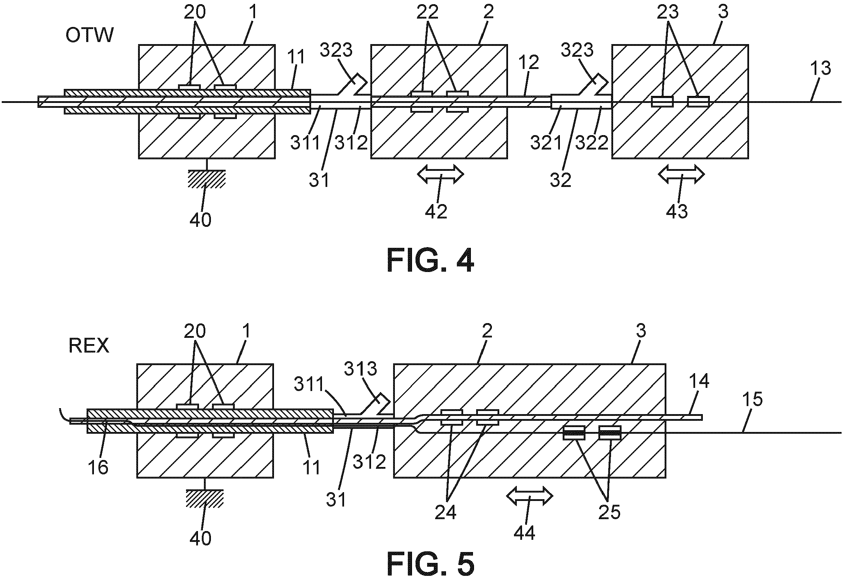

Fig. 4 shows schematically an example of a first embodiment of a catheter robot according to the invention, with an application to an over the wire configuration of flexible elongated medical elements.

Over part of their respective lengths, catheter guide 11 surrounds catheter 12 which itself surrounds guide 13 of catheter. Guide of catheter 13 can slide within catheter 12 which itself can slide within catheter guide 11.

Three translation modules 1, 2 and 3 are successively disposed space apart from one another.

First translation module 1 can translate catheter guide 11 forward and backward, along its own axis, by two pairs of two movable pads 20 respectively facing each other and alternatively clamping catheter guide 11 in order to translate it. The cooperation of these two pairs of two movable pads 20 respectively facing each other and alternatively clamping catheter guide 11 in order to translate it, and which can also rotate this catheter guide 11, is described in more detail in patent application EP 15733825 (hereby incorporated by reference) belonging to the same assignee as present patent application. First translation module is fixed and immobile with respect to base 40 of catheter robot.

Second translation module 2 can translate catheter 12 forward and backward, along its own axis, by two pairs of two movable pads 22 respectively facing each other and alternatively clamping catheter 12 in order to translate it. The cooperation of these two pairs of two movable pads 22 respectively facing each other and alternatively clamping catheter 12 in order to translate it, and which can also rotate this catheter 12, is described in more detail in patent application EP 15733825 (hereby incorporated by reference) belonging to the same assignee as present patent application.

Second translation module 2 can also translate catheter 12 forward and backward, along its own axis, by translating itself, i.e. the whole second translation module 2, on a sliding device 42, which is for example a carrier on wheels or a slider in a rail, in order to follow translation of catheter guide 11. In that case, the two pairs of two movable pads 22 can either add another translation of catheter 12 with respect to first translation module 1, or compensate for translation of catheter 12 coming from the translation of the whole second translation module 2 on its sliding device 42 in order to maintain catheter 12 stationary with respect to first translation module 1 and base 40 of catheter robot.

Third translation module 3 can translate guide of catheter 13 forward and backward, along its own axis, by two pairs of two movable pads 23 respectively facing each other and alternatively clamping guide of catheter 13 in order to translate it. The cooperation of these two pairs of two movable pads 23 respectively facing each other and alternatively clamping guide of catheter 13 in order to translate it, and which can also rotate this guide of catheter 13, is described in more detail in patent application EP 15733825 (hereby incorporated by reference) belonging to the same assignee as present patent application.

Third translation module 3 can also translate guide of catheter 13 forward and backward, along its own axis, by translating itself, i.e. the whole third translation module 3, on a sliding device 43, which is for example a carrier on wheels or a slider in a rail, in order to follow translation of catheter 12. In that case, the two pairs of two movable pads 23 can either add another translation of guide of catheter 13 with respect to second translation module 2 and to base 40, or compensate for translation of guide of catheter 13 coming from the translation of the whole third translation module 3 on its sliding device 43 in order to maintain guide of catheter 13 stationary with respect to second translation module 2 and to base 40 of catheter robot.

The two pairs of two movable pads 20 are aligned with the two pairs of two movable pads 22 which in turn are aligned with the two pairs of two movable pads 23.

A first Y connector or valve 31 is inserted between first translation module 1 and second translation module 2. The first Y connector 31 is fixed to the proximal end of the catheter guide 11 and is fixed to the distal end of the second translation module 2. Catheter 12 surrounding guide 13 of catheter is inserted within first input 312 of first Y connector 31 and comes out of output 311 of first Y connector 31. Output 311 and first input 312 of first Y connector 31 are aligned with common axis of catheter 12 and guide 13 of catheter. A second input 313 of first Y connector 31, disposed at an angle of this common axis of catheter 12 and guide 13 of catheter, for example at 30° or at 45°.

A second Y connector or valve 32 is inserted between second translation module 2 and third translation module 3. The second Y connector 32 is fixed to the proximal end of the catheter 12 and is fixed to the distal end of the third translation module 3. Guide 13 of catheter is inserted within first input 322 of second Y connector 32 and comes out of output 321 of second Y connector 32. Output 321 and first input 322 of second Y connector 32 are aligned with axis of guide 13 of catheter. A second input 323 of second Y connector 32, disposed at an angle of this axis of guide 13 of catheter, for example at 30° or at 45°.

In a working embodiment, when the pads 20 of the first translation module 1 move in order to translate the catheter guide 11, the second translation module 2 and the third translation module 3 have to be translated by the same amount along the common axis of the catheter 12 and of the guide 13 of catheter. When the pads 22 of the second translation module 2 move in order to translate the catheter 12, the third translation module 3 has to be translated by the same amount along the common axis of the catheter 12 and of the guide 13 of catheter. This ensures that all movable translation modules, i.e. second translation module 2 and third translation module 3, will be always located at their respective most possible distal positions, and thus will allow to avoid the negative bending effect of guide 13 of catheter shown on figure 3.

Another solution, to avoid this negative bending effect of guide 13 of catheter or (here) guidewire 13, could be thought of. It would consist in allowing for the considered translation module not to be at the most possible distal position and providing for a guiding channel between this translation module and the closest more distal device (for example, the closest more distal device to the guidewire 13 is the Y valve 32 and then the catheter 12). Such devices can be used in other catheter robots, including for example a flexible track, as described in detail for example in the patent application US2018193603. In this case, a guidewire 13 is pushed from its proximal end by a catheter robot, and this flexible track avoids any bending of this guidewire 13 in its portion located between the catheter robot and the introducer (which is, in this case, the closest more distal device). This flexible track plays the role of a king of "guiding tunnel" with a variable length. This variable length is then needed to accommodate for the variable distance between the manipulation location of a manipulated flexible elongated medical element, i.e. the location of the translation modules that manipulate it, and the position where the manipulated flexible elongated medical element is coaxially inserted into another flexible elongated medical element.

The benefit of some embodiments of the invention is, among others, to avoid this flexible track, or any other equivalent burdensome solution, because this flexible track is necessarily part of the consumable, and as such, it adds complexity to the consumable. This can lead to, for instance, reduced usability, including risk of manipulation errors, risk of reduced reliability, higher cost ...

Embodiments of the invention described here solve this problem, because the push movement, i.e. translation from a proximal to a distal position, is done at a place where the flexible elongated medical element enters another flexible elongated medical element (as explained above), the said other flexible elongated medical element acting as a guiding channel, and thus suppressing the need for this burdensome flexible track or any other added equivalent burdensome mechanism. This has been made possible, because the translation is obtained using the internal mechanism of the translation module (using for instance small repeated steps as described in patent application FR3022147, hereby incorporated by reference). This is different from the principle used in patent application US2018193603, where the proximal end of a flexible elongated medical element (in this case a guidewire for example) is attached to a translation module, and then the whole translation motion performed by the translation module is used to generate the same translation of the flexible elongated medical element.

Indeed, embodiments of the invention can avoid using a burdensome flexible track (or any equivalent burdensome guiding principle), thanks to the combination of both:
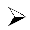 First, placing the translation module at the most distal position possible, so that the flexible elongated medical element going out of the translation module from its distal end directly enters coaxially in another flexible elongated medical element (a catheter with a higher diameter, a Y valve, an introducer, etc.), this another flexible elongated medical element acting as a guiding channel,
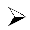 and second, ensuring the motion of the flexible elongated medical element using the internal mechanisms of the translation module, where the flexible elongated medical element moves relatively to the translation module rather than performing a global motion of the translation module where the flexible elongated medical element and the translation module would be linked together and thus would be compelled to move together at exactly the same speed.

The architecture shown in figure 4 includes a total of 3 translation modules 1, 2 and 3, robotically translating 3 flexible elongated medical elements, 11, 12 and 13. However, in some cases, it may be desirable to use even more flexible elongated medical elements. The architecture disclosed on figure 4 can be extrapolated to a four translation modules architecture or even more. The most distal module is fixed relatively to the patient, or more precisely to the patient bed, whereas all other modules can be translated along the axis of the flexible elongated medical elements.

Fig. 5 shows schematically an example of a second embodiment of a catheter robot according to the invention, with an application to a rapid exchange configuration of flexible elongated medical elements.

Over a very short part 16 of catheter 14 length, catheter guide 11 surrounds catheter 14 which itself surrounds guide 15 of catheter. Guide of catheter 15 can slide within catheter 14 which itself can slide within catheter guide 11, over this very short part 16 of catheter 14.

Three translation modules 1, 2 and 3 are successively disposed space apart from one another.

First translation module 1 can translate catheter guide 11 forward and backward, along its own axis, by two pairs of two movable pads 20 respectively facing each other and alternatively clamping catheter guide 11 in order to translate it. The cooperation of these two pairs of two movable pads 20 respectively facing each other and alternatively clamping catheter guide 11 in order to translate it, and which can also rotate this catheter guide 11, is described in more detail in patent application EP 15733825 (hereby incorporated by reference) belonging to the same assignee as present patent application. First translation module is fixed and immobile with respect to base 40 of catheter robot.

Second translation module 2 can translate catheter 14 forward and backward, along its own axis, by two pairs of two movable pads 24 respectively facing each other and alternatively clamping catheter 14 in order to translate it. The cooperation of these two pairs of two movable pads 24 respectively facing each other and alternatively clamping catheter 14 in order to translate it, and which can also rotate this catheter 14, is described in more detail in patent application EP 15733825 (hereby incorporated by reference) belonging to the same assignee as present patent application.

Third translation module 3 can translate guide of catheter 15 forward and backward, along its own axis, by two pairs of two movable pads 25 respectively facing each other and alternatively clamping guide of catheter 15 in order to translate it. The cooperation of these two pairs of two movable pads 25 respectively facing each other and alternatively clamping guide of catheter 15 in order to translate it, and which can also rotate this guide of catheter 15, is described in more detail in patent application EP 15733825 (hereby incorporated by reference) belonging to the same assignee as present patent application. Except on very short part 16, where catheter 14 and guide 15 of catheter are coaxial with each other, catheter 14 and guide 15 of catheter are outside from each other and parallel to each other. Therefore, the two pairs of two movable pads 24 are transversely shifted from the two pairs of two movable pads 25.

Second translation module 2 and third translation module 3 are solidarized with each other and can only translate themselves together with complete synchronization to each other. Indeed, both second translation module 2 and third translation module 3 can translate themselves together on a common sliding device 44, which is for example a carrier on wheels or a slider in a rail, in order to follow translation of catheter guide 11. Second translation module 2 and third translation module 3 can also translate respectively catheter 14 and guide 15 of catheter forward and backward, along their own respective axis, by translating themselves together.

In that case, the two pairs of two movable pads 24 can either add another translation of catheter 14 with respect to first translation module 1, or compensate for translation of catheter 14 coming from the translation of both second translation module 2 and third translation module on their common sliding device 44, in order to maintain catheter 14 stationary with respect to first translation module 1 and base 40 of catheter robot.

In that case, the two pairs of two movable pads 25 can either add another translation of guide 15 of catheter with respect to first translation module 1, or compensate for translation of guide 15 of catheter coming from the translation of the both second translation module 2 and third translation module 3 on their common sliding device 44 in order to maintain guide 15 of catheter stationary with respect to first translation module 1 and base 40 of catheter robot.

A first Y connector or valve 31 is inserted between first translation module 1 and second translation module 2 linked to third translation module 3. First Y connector 31 is fixed to the proximal end of the catheter guide 11 and is fixed to the distal end of the second translation module 2. Catheter 14 and guide 13 of catheter parallel to each other, are inserted within first input 312 of first Y connector 31 and comes out of output 311 of first Y connector 31. Output 311 and first input 312 of first Y connector 31 are aligned with common axis of catheter 14 and guide 15 of catheter. A second input 313 of first Y connector 31, disposed at an angle of the two parallel axes respectively of catheter 14 and guide 15 of catheter, for example at 30° or at 45°.

The invention has been described with reference to preferred embodiments. However, many variations are possible within the scope of the invention.

## Claims

1. Catheter robot comprising:
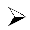 a base (40),
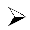 at least 3 catheter translation modules (1, 2, 3) supported by said base (40),
∘ each catheter translation module (1, 2, 3) comprising two pairs of movable pads (20, 22, 23, 24, 25):
▪ said pads of a same pair at least partly facing each other,
▪ alternatively, each of said two pairs clamping between its pads a flexible elongated medical element (11, 12, 13, 14, 15) so as to then translate longitudinally said flexible elongated medical element,
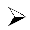 said catheter translation modules (1, 2, 3) being successively longitudinally disposed so that:
∘ first catheter translation module (1) has its pairs of pads (20) performing a longitudinal translation of a first flexible elongated medical element which is a catheter guide (11),
∘ second catheter translation module (2):
▪ can translate longitudinally with respect to said first catheter translation module (1),
▪ its pairs of pads (22, 24) performing a longitudinal translation of a second flexible elongated medical element which is a catheter (12, 14), preferably a catheter balloon or a catheter stent,
∘ third catheter translation module (3):
▪ can translate longitudinally with respect to said first catheter translation module (1),
▪ its pairs of pads (23, 25) performing a longitudinal translation of a third flexible elongated medical element which is a guide (13, 15) of said catheter.

2. Catheter robot according to claim 1, wherein the catheter robot comprises a control unit configured for commanding the translation of the second catheter translation module (2) by following the translation of the first flexible elongated medical element (11), and for controlling the translation of the second flexible elongated medical element (12) by compensating the translation of the second catheter translation module (2) so as to maintain the second flexible elongated medical element (12) stationary with respect to said base (40).

3. Catheter robot according to any of preceding claims, wherein said catheter translation modules (1, 2, 3) are all structurally identical.

4. Catheter robot according to any of preceding claims, wherein said catheter translation modules (1, 2, 3) are successively longitudinally spaced apart from one another.

5. Catheter robot according to any of preceding claims, wherein said third catheter translation module (3) can translate longitudinally with respect both to said first catheter translation module (1) and to said second catheter translation module (2).

6. Catheter robot according to claim 5, wherein:
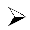 a first Y connector (31) is located between said first (1) and second (2) catheter translation modules, one (312) of Y branches and output (311) of Y being disposed longitudinally,
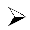 a second Y connector (32) is located between said second (2) and third (3) catheter translation modules, one (322) of Y branches and output (321) of Y being disposed longitudinally.

7. Catheter robot according to any of preceding claims, wherein said guide (13) of catheter translates within said catheter (12) itself translating within said catheter guide (11), for at least part of their respective lengths.

8. Catheter robot according to any of preceding claims, wherein the catheter robot comprises a control unit configured for commanding the translation of the third catheter translation module (3) by following the translation of the second flexible elongated medical element (12), and for controlling the translation of the third flexible elongated medical element (13) by compensating the translation of the third catheter translation module (3) so as to maintain the third flexible elongated medical element (13) stationary with respect to said base (40).

9. Catheter robot according to claim 1 or 2, wherein said second (2) and third (3) catheter translation modules are fastened together so as to be able to translate longitudinally with respect to said first catheter translation module (1) only jointly.

10. Catheter robot according to claim 9, wherein said pairs of pads (25) of said third catheter translation module (3) are transversely shifted from said pairs of pads (24) of said second catheter translation module (2).

11. Catheter robot according to claim 10, wherein:
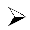 said pairs of pads (25) of said third catheter translation module (3) are transversely shifted from said pairs of pads (24) of said second catheter translation module (2), so that:
∘ said guide (15) of catheter and said catheter (14) can translate:
▪ parallel to each other over the length extending along said 3 catheter translation modules (1, 2, 3),
▪ along each other for most of the length of said catheter (14),
▪ both within said catheter guide (11) for at least part of their respective lengths,
∘ said guide (15) of catheter translating within said catheter (14) for only distal portions (16) of their respective lengths.

12. Catheter robot according to any of preceding claims, wherein said first catheter translation module (1) is fixed with respect to said base (40).

13. Catheter robot according to any of preceding claims, wherein said base (40) is an end of an articulated robotic arm.

14. Catheter robot according to any of preceding claims, wherein said catheter translation modules (1, 2, 3) are successively longitudinally disposed from one another, in an order which is: first (1) then second (2) and then third (3) catheter translation module.

15. Catheter robot according to any of preceding claims, wherein it comprises: at least 4 catheter translation modules supported by said base (40), which are successively longitudinally disposed, or preferably at least 5 catheter translation modules supported by said base (40), which are successively longitudinally disposed, and more preferably only 5 catheter translation modules.

16. Catheter robot according to claim 15, wherein: second and third catheter translation modules are configured as rapid exchange catheter translation modules, and fourth and fifth catheter translation modules are configured as over the wire catheter translation modules, or all second to fifth catheter translation modules are configured as over the wire catheter translation modules.

17. Catheter robot according to any of preceding claims, wherein each catheter translation module (1, 2, 3) is also a catheter rotation module alternatively, each of said two pairs clamping between its facing pads (20, 22, 23, 24, 25) a flexible elongated medical element (11, 12, 13, 14, 15) so as to then rotate around longitudinal axis said flexible elongated medical element, by making said flexible elongated medical element rolling between its facing pads.
